# EUROPEAN PATENT SPECIFICATION

(11) **EP 2 158 560 B1**
(45) Date of publication and mention of the grant of the patent: **22.09.2010**
(21) Application number: 08826717.4
(22) Date of filing: 09.06.2008
(51) Int. Cl.: G06K 7/015, G06K 7/08, G06K 19/067, A61M 5/24, A61M 5/28, A61M 5/315

(54) **CONTACT FREE READING OF RESERVOIR IDENTIFICATION CODES**
KONTAKTFREIE ABLESUNG VON SPEICHERIDENTIFIKATIONSCODES
LECTURE SANS CONTACT DE CODES D'IDENTIFICATION D'UN RÉSERVOIR

(30) Priority: 09.06.2007 EP 07109943
(43) Date of publication of application: 03.03.2010
(73) Proprietor: Novo Nordisk A/S, 2880 Bagsværd (DK)
(72) Inventor: NIELSEN, Søren, Kragh, DK-4340 Tølløse (DK); BERGGREN, Bo, Erik, S-22736 Lund (SE); VEJGAARD-NIELSEN, Jeppe, DK-3400 Hillerød (DK)
(86) International application number: PCT/EP2008/057185
(87) International publication number: WO 2009/015933

(56) References cited:
- EP-A2- 0 248 165
- WO-A-2005/089835
- WO-A-2007/107562

## Description

### FIELD OF THE INVENTION

The present invention provides an alternative arrangement and method for contact free reading of Identification codes on medicament containing reservoirs, such as cartridges or flexible reservoirs. In particular, the present invention relates to a medicament containing reservoir for a medication delivery device. The reservoir has an identification code arranged on an exterior surface thereof. This identification code comprises a plurality of electrode elements adapted to Interact with a transmitter/receiver arrangement arranged on an associated medication delivery device.

### BACKGROUND OF THE INVENTION

WO 01/84542 discloses a cartridge for a medication delivery device, the cartridge carrying an identification code represented by a number of bars. The bars are arranged perpendicular to the axis of the cartridge. Each bar is along its entire length provided with an optical grating which diffracts and reflects light impinging the surface carrying the Identification code. In this way a minor part of the impinging light is reflected from the surface of the bar as a set of light beams of which beams at least one is detected for the Indication of the presence of the bar when said bar passes a reading light field. The reflections from the bars may be interpreted as representing "1"s and "0"s in a binary code. Thus, in order to read the identification codes suggested In WO 01/84542 a sophisticated optical detection system is required.

US 6,110,152 discloses a cartridge for containing a fluid and for use with an electronic delivery device. The cartridge of US 6,110,152 includes a housing for holding the fluid and an information providing source. The information providing source is coupled to the cartridge housing to operatively couple with the electronic delivery device to provide predetermined information regarding the cartridge to the electronic delivery device. For example, the information providing source may be a set of wires and contacts, or contact bands, that provide the predetermined Information to the electronic delivery device by producing a binary code. Alternatively, the information providing source is a bar code that provides the predetermined information to electronic delivery device by reading of the bar code. The cartridge may be used in a system that includes an electronic delivery device, such as an electronic pen-type Injector and/or Infusion pump. It should be noted that in order to read the information providing source on the cartridge an electrical connection should be provided between the information providing source and the electronic delivery device.

WO 2004/084795 relates to marking of cartridges or similar devices. The marking can be In an electronically readable form while being transparent. The transparent conductor can be In form of a polymer, an ITO and the like. Similar to US 6,110,152 electrical connections need to be established between the transparent conductors and an associated medication delivery device In order to read the marking.

In WO 2005/089835 RFID coded markers are disclosed in connection with infusion pumps.

Further, In WO 2007/107562, non-contact based reading of reservoir identification codings are disclosed. As identified therein US 4,420,754 discloses a system for measuring the relative movement between two elements, such as the scale and slide of a hand-held measuring Instrument. The system according to US 4,420,754 Includes the provision of a number of groups of supply electrodes on the slide. Each of these electrodes in each group being supplied from a respective one of a multiple number of output signals from a signal generator so that all of the supply electrodes are furnished with voltages according to a cyclic pattern. The slide also has at least one receiving electrode which feeds a signal processing unit. The scale is provided with an electronic pattern comprising internally galvanically connected parts, one being a detecting part, located close to the area where the supply electrodes of the slide are moved, the other of the two parts being a transferring part which is located close to the area where the receiving electrode of the slide is moved. The movement of the slide along the scale generates a signal from the receiving electrode which is derived from the signals from at least two adjacent supply electrodes and the position of the slide is determined by a signal processing unit which identifies the amplitude ratio of the received signals. Thus, the system suggested In US 4,420,754 relates to an arrangement for detecting relative movements between two elements.

It is an object of the present invention to provide a fail-safe and cost-effective solution involving an identification code In form of electrode elements arranged on a medicament reservoir, said identification code being readable by an arrangement of transmitters and receivers arranged on an associated medication delivery device.

It is an advantage of the present invention that the Identification code provided on medicament containing cartridges may be easily readable by contact free means. Thus, no sophisticated detection systems such as optical detection systems are required.

A further advantage of the present invention is that the coding system provides a fail-safe reading of each code, irrespective of the mutual orientation of the coded Information provided on a medicament reservoir with respect to the reading heads of the associated medical delivery device. In particular, the present invention provides for compensation for a possible skew angle between the coded information and the reading heads of the device. This again provides for a larger number of distinct codes which can be safely distinguished by the medical delivery device.

### SUMMARY OF THE INVENTION

The above-mentioned object is complied with by providing, In a first aspect a medicament reservoir for a medical delivery device adapted to receive the medicament reservoir and adapted to capacitively couple to and Identify an identification code arranged on the medicament reservoir when received by the medical delivery device. The reservoir identification code comprises a first array of discrete electrodes having an equally spaced pitch P and forming a receiver array, a second array of discrete electrodes having an equally spaced pitch P and forming a first transmitter array, wherein the receiver array and the first transmitter array are arranged substantially parallel and extending along a first direction. The respective electrodes of the receiver array are electrically connected to the respective electrodes of the first transmitter array. Further, the electrodes of the first transmitter array are either aligned or offset along the first direction with respect to respective electrodes of the receiver array by a first offset value, the magnitude of the first offset value being dedicated the type of medicament reservoir. A further third array of discrete electrodes having an equally spaced pitch P forms a second transmitter array which is arranged substantially parallel to the first transmitter array. The respective electrodes of the second transmitter array are electrically connected to respective electrodes of the first transmitter array. The electrodes of the second transmitter array are either aligned or offset along the first direction with respect to respective electrodes of the first transmitter array. By providing two transmitter arrays on the reservoir, each of which may be read by a dedicated reading head of an associated medical delivery device, the correct reading of a large number of distinct codes is enabled.

The electrodes forming the three arrays may be made of an electrically conducting material such as an optically transparent or non-transparent material. The electrodes within each array are galvanically separated. Thus, only corresponding receiver and transmitter electrodes are galvanically connected.

In one embodiment, the first transmitter array is arranged between the receiver array and the second transmitter array. In other embodiments, the receiver array is arranged between the first and the second transmitter array.

According to some embodiments, the electrodes of the second transmitter array are offset along the first direction with respect to respective electrodes of the first transmitter array. According to other embodiments, the electrodes of the second transmitter array are aligned along the first direction with respect to respective electrodes of the receiver array, that is, the offset along the first direction of the second transmitter array with respect to the receiver array is zero. In this way, an identification of possible mis-alignment between the reservoir identification code and the corresponding reader sensor circuitry is readily obtainable.

In still other embodiments, the electrodes of the second transmitter array are aligned along the first direction with respect to respective electrodes of the first transmitter array.

In further embodiments, the position of the electrodes of the second transmitter array along the first direction with respect to respective electrodes of the receiver array defines a second offset value, the combination of the first and second offset values being dedicated the type of medicament reservoir. Such coded reservoirs provide a larger number of distinct codes which can be safely distinguished by an associated medical delivery device. If real-life possible skew angles between the device reader sensor electronics and the identification code is limited to small known angles, the degree of offset of the second transmitter array may be used both as a measure of degree of alignment and as an additional level of coding, I.e. to provide a second level of coding in addition to the coding obtained by the first transmitter array. One pre-requisite for this use is that the distinct offset values of the second offset value is larger than any effect attributable to a possible mis-aligment.

To reduce mechanical wear, the electrode arrays may be at least partly covered by a layer of dielectric material.

The reservoir may be provided as a cartridge comprising a rear end and a front end, the front end comprising an arrangement for securing a fluid conduit such as an injection needle to the cartridge - optionally via a cartridge holder into which the cartridge is adapted to be inserted. The electrode arrays may be arranged near the rear end of the cartridge so that the plurality of electrode elements have a spatial overlap with a displaceable rubber piston when said piston is in its initial position.

Each of the electrode arrays may form an essentially periodic pattern, the direction of periodicity being substantially perpendicular to an axial direction of the cartridge. Preferably, the essentially periodic pattern forms an essentially unbroken path around the exterior surface part of the cartridge whereby the identification of the cartridge becomes Independent of the angular orientation of the cartridge when Inserted Into the medication delivery device.

As previously mentioned, the electrode arrays may be made of an optically transparent material. By applying optically transparent electrodes the electrodes are allowed to occupy more space on the cartridge. In case of optically transparent electrode arrays the cartridge may have an axial direction, wherein the electrode arrays form an essentially periodic pattern, the direction of periodicity being substantially parallel to the axial direction of the cartridge.

The electrode arrays, transparent or non-transparent, may be arranged on a flexible foil, said foil being secured to the exterior surface part of the cartridge using an adhesive between the foil and the exterior surface part.

The electrode arrays may be adapted to couple to one or more transmitters and to one or more receivers arranged on the associated medication delivery device In a preferably capacitive manner. However, coupling between electrode elements and transmitters/receivers may also be accomplished by inductive means.

Alternatively, the medicament reservoir may be of a kind different from cartridges. Thus, the reservoir may comprise one or more collapsible sidewall portions, said one or more collapsible sidewall portions being adapted to collapse during emptying of the reservoir.

In a second aspect, the present invention relates to a medical delivery device according to claim 8. It is adapted to couple to or receive the medicament reservoir according the first aspect where the medicament reservoir comprises electrodes for respectively forming a receiver array, a first transmitter array and a second transmitter array, said arrays arranged substantially parallel along a first direction to define a reservoir identification code. The electrodes within each of the three arrays are equally spaced having substantially the same pitch P. Respective electrodes of the receiver array, the first transmitter array and the second transmitter array are mutually connected by placing connecting electrodes therebetween. The medical delivery device comprises:
- a group of device transmitter electrodes of equally spaced pitch P arranged along a second direction and adapted to generally align with and capacitively couple to a respective group of electrodes of the receiver array disposed on the reservoir,
- a first reader head having electrodes for capacitively coupling to a respective group of electrodes of the first transmitter array disposed on the reservoir,
- signal generator means for applying a plurality of input signals to respective electrodes of said group of transmitter electrodes, and
- electric circuitry adapted to receive signals detected by the first reader head to detect the magnitude of a third offset value representing the degree of offset of electrodes of the first transmitter array along the second direction with respect to electrodes of the receiver array.

The medical delivery device further comprises a second reader head having electrodes for capacitively coupling to a respective group of electrodes of the second transmitter array disposed on the reservoir. The electric circuitry is further adapted to receive signals from the second reader head to detect the magnitude of a fourth offset value representing the degree of offset of electrodes of the second transmitter array along the second direction with respect to electrodes of the receiver array and to identify the reservoir identification code on the basis of the third and fourth offset values.

In one embodiment, the electric circuitry of the medical delivery device is adapted to detect a skew angle defined by the angle between the first direction and the second direction based upon the detected fourth offset value.

In a further embodiment, the medical delivery device further comprises alarm means coupled to the electric circuitry and where the electric circuitry is adapted to generate an alarm if a detected skew angle is larger than a pre-defined value. Hereby, a user of the device may be informed that no proper reading of the identification code has been obtained.

In a still further embodiment, the electric circuitry is adapted to calculate a modified code offset value based upon the detected third offset value and compensated by the detected skew angle.

In a still further embodiment, the medical delivery device may be adapted to identify an Identification code in accordance with the combination of the detected third and fourth offset values, in order to obtain a large number of distinct identification codes.

In one embodiment of the present invention four electrical Input signals are applied to four transmitters arranged on the associated medication delivery. These four electrical Input signals are approximately 90 degrees out of phase so as to form a quadrature electrical Input signal. Each electrical input signal may oscillate with a frequency within the range 50 kHz - 150 kHz, such as within the range 90 kHz - 110 kHz. However, other frequency ranges may also be applicable.

In a third aspect, the present invention relates to a medication delivery device comprising a medicament reservoir according to the first aspect of the present invention.

In a fourth aspect, the present invention relates to a flexible foil as defined in claim 14. The flexible foil is adapted to be arranged on an exterior surface part of a medicament reservoir to thereby constitute a medicament reservoir according to the first aspect.

In accordance with the present invention, a medication delivery system comprising at least one medical delivery device according to the second aspect, and one or more mutually distinct medicament reservoirs according to the first aspect may be provided. In such system, the or each delivery device is adapted to identify the identification codes of the medicament reservoirs, and to present an indication in response to an allowed medicament reservoir received by the medical delivery device, or alternatively or in addition, to present an indication in response to a not allowed medicament reservoir received by the medical delivery device.

### BRIEF DESCRIPTION OF THE DRAWINGS

The present invention will now be explained in further details with reference to the accompanying figures, wherein
Fig. 1 shows a traditional digital calliper scale,
Fig. 2 shows discrete portions of a calliper scale,
Fig. 3 illustrates a code phase shift,
Fig. 4 shows a reader head and driver signals,
Fig. 5 illustrates driver signals and detected signal for code "0",
Fig. 6 illustrates driver signals and detected signal for code "n",
Fig. 7 shows driver head and receiver/transmitter elements,
Fig. 8 shows code "0" and code "n" arranged on a prior art cartridge,
Fig. 9 shows multiple reader heads arranged In a prior art device for use with a prior art cartridge,
Fig. 10 shows an alternative implementation of electrode elements according to a prior art system,
Fig. 11a-e show an identification code and a reader head assembly providing compensation for skewed labels, and
Fig. 12 shows an embodiment of the invention.

While the invention is susceptible to various modifications and alternative forms, specific embodiments have been shown by way of example in the drawings and will be described In detail herein. It should be understood, however, that the invention Is not intended to be limited to the particular forms disclosed. Rather, the invention is to cover all modifications, equivalents, and alternatives falling within the spirit and scope of the invention as defined by the appended claims.

### DETAILED DESCRIPTION OF THE INVENTION

In its most general aspect the present invention relates to a medicament containing reservoir having electrode elements positioned on an exterior surface thereof. The medicament containing reservoir can be a traditional cartridge, a flexible reservoir with collapsible sidewall portions or the like. The electrode elements can be made of a transparent or non-transparent material. Suitable transparent materials are for example electrically conducting polymers or ITOs. Non-transparent electrode elements can be formed as a metallic pad or a pattern of metallic pads adapted to couple to a number of transmitters and a number of receivers arranged on an associated medication delivery device. Electrode elements of transparent or non-transparent nano-tubes can also be applied. The coupling between electrode elements and transmitters/receivers can be capacitive.

The overall principle of detection between electrode elements and a reader head comprising the transmitter/receiver arrangement is similar that disclosed In US 4,420,754, US 4,878,013 and EP-A-248,165. However, in the present invention discrete values of the scale shown in said three references are associated with the various types of medicament. Thus, to a certain type of medicament a single and well-defined value is associated.

In the following the notation "cartridge" will be used only. However, It should be noted that the term cartridge should be interpreted broadly, thus covering both traditional cartridges and for example reservoirs having flexible or collapsible sidewalls. By determining a capacitive or Inductive coupling between electrode elements of the cartridge and transmitter/receivers the identification code of the cartridge may be determined In a very simple and contact free manner. When the identification code of the cartridge Is determined, the type of medicament contained in the cartridge is determined in an unambiguous manner. However, not only the type of medicament in the cartridge can be determined by the contact free coupling between electrode elements and the transmitter/receiver arrangement. Also data relating to fabrication date, batch number, number of available doses etc. may be embedded in the coding for subsequently identification in the associated medical delivery device. Further, any other parameter relating to the use of the cartridge may be dedicated one of a plurality of different identification codes.

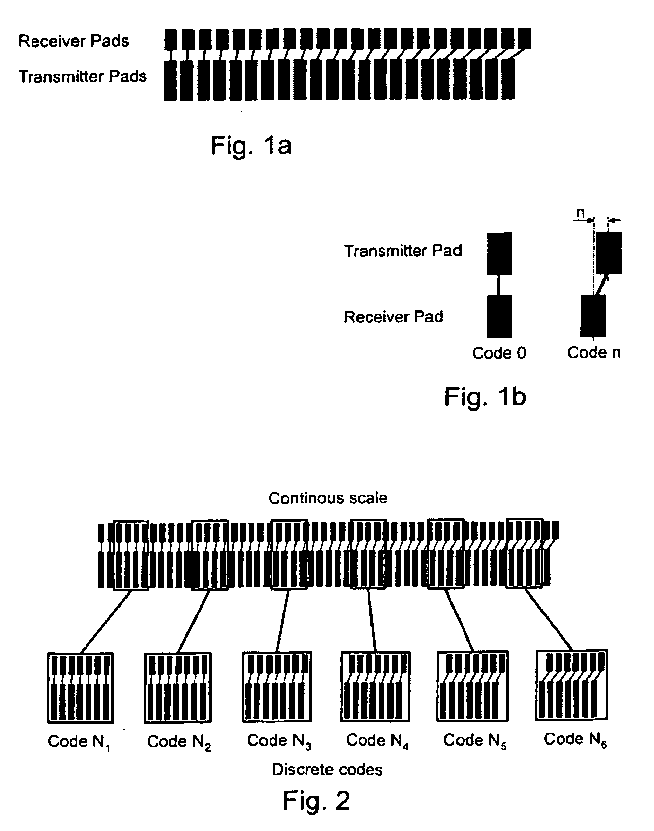

Referring now to Fig. 1a and 1b the means for deriving codes for marking cartridges are based on an absolute position measuring principle commonly used in digital callipers. The display part of a digital calliper contains the reader head whereas the scale part of the calliper holds the scale. The scale consists of two rows of metallised pads where one row - the transmitter pads - have pads equidistantly placed, and the row above - the receiver row - have pads that are also equidistantly placed but at a somewhat greater distance from each other. In this way a continuous scale is produced where the displacement of a receiver pad In relation to its connected transmitter pad in any given position along the scale is a direct measure of its absolute position on the scale. By using discrete parts of a code like the one used in some digital callipers, see Fig. 1b, a code, readable by non-contacting means, is created.

As depicted in Fig. 2, the coding method known from WO 2007/107562 and useable with the present invention is based on taking snippets of a continuous scale and using these as discrete codes representing the various cartridges and the drugs contained within. By spacing these snippets carefully the signal-to-noise ratio, or rather the probability of the right code read, is increased. On the other hand, the greater the safety margin between adjacent codes, the fewer the available codes.

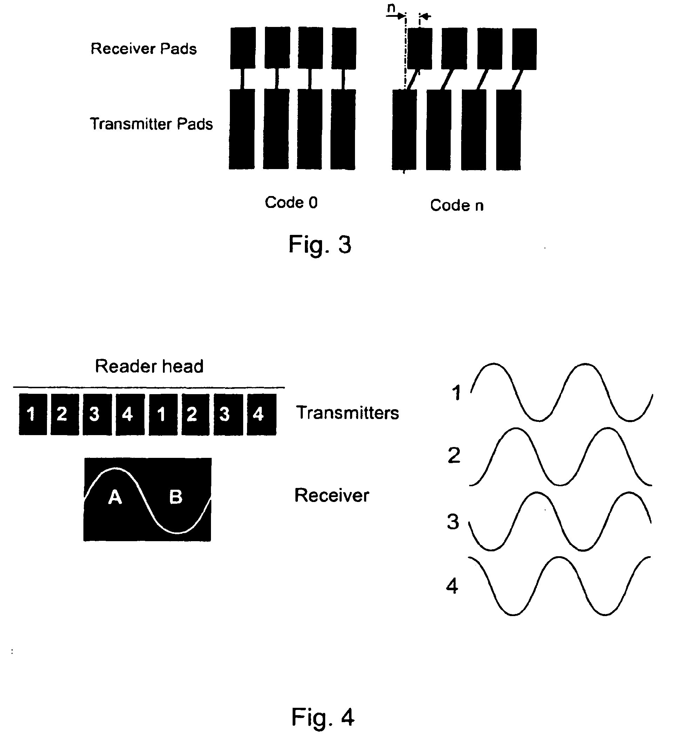

The displacement of the receiver pads to their transmitter counterparts, denoted "n" in Fig. 3, is measured by a reader head that capacitively couples four sinusoidal signals in quadrature to four receiver pads on the code. The signals received are passed on to the connected transmitter pads on the code and in turn transmitted back to the receiver part of the reader head. The amplitude ratios of the received quadrature signals give a resulting sinusoidal composite signal whose phase relative to the transmitted signals from the reader head is a direct measure of the code value, and thereby the type of the medicament cartridge.

Some simplified examples to explain the quadrature signal summation and phase shift measurements which form the basis of the digital calliper principle are depicted In Figs. 4-6. The reader head Is depicted In Fig. 4, as are the four sinusoidal quadrature signals driving the transmitters. Note the 90° phase shift between the transmitted signals - which is the definition of quadrature. This modulation scheme - in conjunction with synchronous demodulation and phase angle measurement by waveform zero crossing time differences In the reader head ASIC - makes for a very stable output value despite the very small capacitances involved. The sinusoidally shaped receiver electrodes of the reader head weight the received signals in order to minimise reading disturbances when moving from one code pad to the next. This Is similar to when a digital calliper is adjusted.

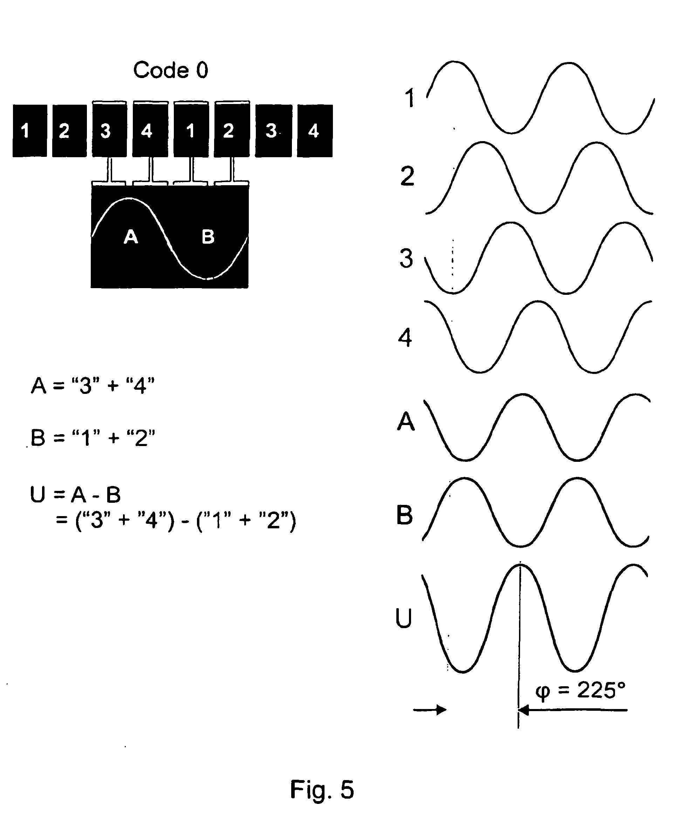

When the reader head is applied to a code with code value 0, which means that 'n' is zero in Fig. 3, the resulting waveforms are as illustrated In Fig. 5. As seen the signals from receivers A and B are 180 degrees out of phase. Compared to transmitter signal "1" a phase shift, ϕ, of 225 degrees is detected. Thus, Code 0 yields a phase shift value of 225° compared to transmitter signal "1" and the output signal U from the reader head electronics is derived from the *difference* between signals A and B. In this way any external noise signals that may have entered the reader head are cancelled while the output signal is doubled in amplitude. Horizontally offsetting or shifting the code relative to the reader head will, of course, still yield the same phase value from the reader head.

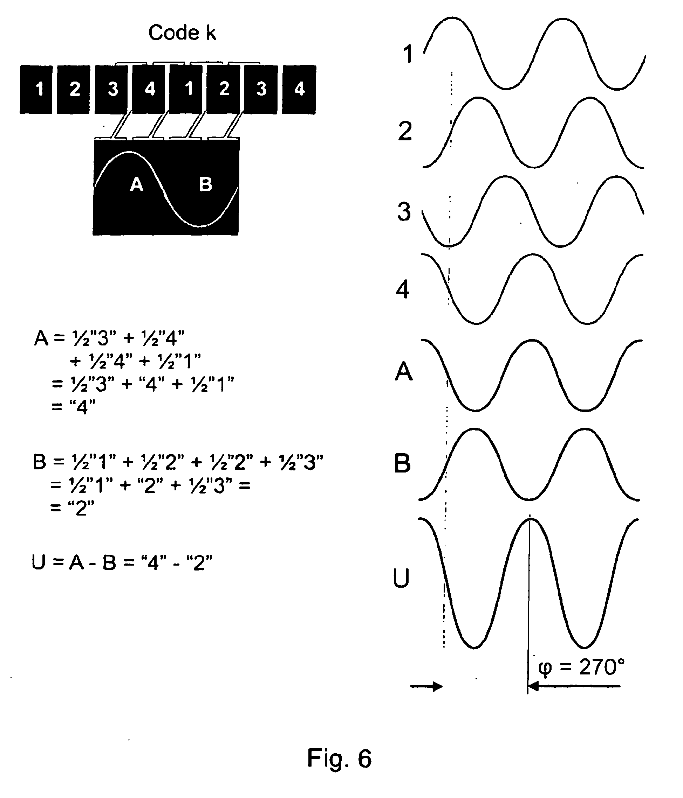

In another example shown in WO 2007/107562, see Fig. 6, the reader head is applied to a code with code value k. In this case the transmitter pads of the reader head are *not* aligned with the receiver pads of the code. Instead two adjacent transmitter pads transmit to one receiver pad on the code. The waveforms are also illustrated in Fig. 6. Note that in the equations of these examples small terms that will cancel out in the signal processing have been eliminated. Also, the signal weighting of the reader pad shape Is simplified to the weights 0, ½ or 1. A comprehensive explanation of the working principle can be found in for example US 4,420,754.

Thus, the coding method useable with the present invention involves placing a number of arrays of discrete electrically conducting receiver pads on the medical container, such as a cartridge. Each of these receiver pads are connected to a respective transmitter pad In an array of discrete transmitter pads. The lateral displacement of the transmitter pad array to its receiver counterpart Is the coded value. This value is measured by a reader head that by non-contacting, capacitive means transmits signals to the receiver pads on the medical container and receives, also by non-contacting, capacitive means, signal that is changed In proportion to the lateral displacement of the pad arrays on the medical container. This signal change is decoded by the electronics associated with the reader head and output as a digital code value.

In Figs. 4-6 the number of transmitters is eight. It should be noted that the number of transmitters can be different from eight (both higher and lower). Also, other types of drive signals (other than quadrature drive signals) can be applied to the transmitters. For example, six drive signals shifted 60 degrees can be applied to six transmitters. The receiver can also be implemented differently than depicted In Figs. 4-6. For example, the receiver can be implemented with additional periods than the one shown In Figs. 4-6. Also, the shape of the receiver can be different from sinusoidal - for example quadratic, rectangular and triangular are also applicable shapes. The only requirement is that the receiver requires at least two receiver pads arranged 180 degrees out of phase.

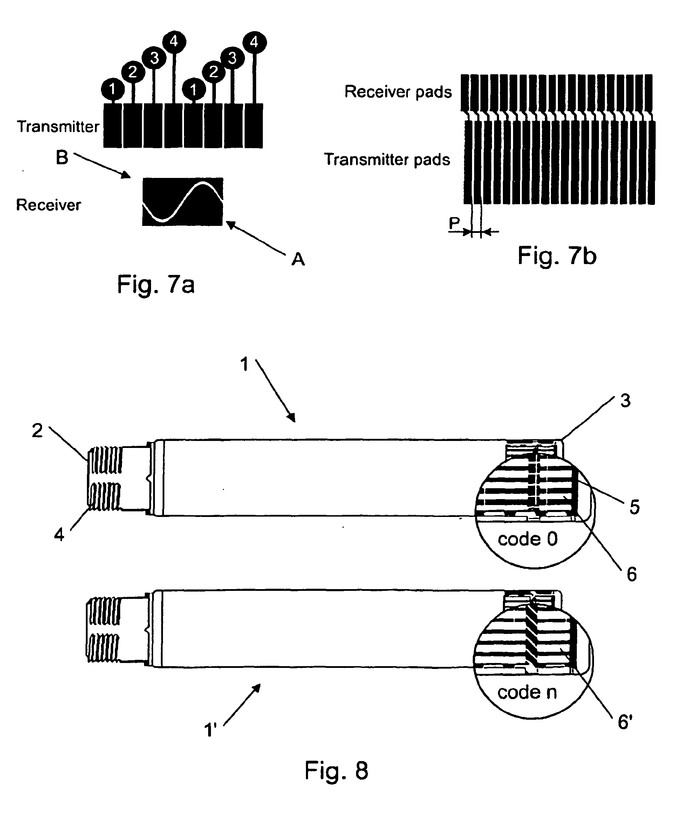

Again referring to WO 2007/107562, Fig. 7a shows a reader head whereas Fig. 7b shows an identification code comprising a first transmitter array comprising a series of electrodes denoted "transmitter pads" and a receiver array comprising a series of electrodes denoted "receiver pads". As depicted in Fig. 7b a constant lateral displacement exists between the receiver pads and the transmitters pads. In this way both the receiver array and the transmitter array forms pads where the pitch "P" between pads in each of the said arrays are substantially the same and substantially constant along the entire extension of each array. Now referring to Fig. 7a the reader head of the device comprises a total of eight transmitter electrodes, where transmitters "1" are driven in parallel. Similarly, transmitters "2", "3" and "4", respectively, are driven in parallel. The receiver of the reader head comprises two receiver electrodes "A" and "B".

Thus, in accordance with this prior art embodiment, the identification code is provided as a first array of discrete electrodes forming a receiver array and a second array of discrete electrodes forming a first transmitter array where the two arrays are arranged substantially parallel, each array extending along a first direction. The notion "discrete electrodes" means that each electrode within each of the receiver array and the first transmitter array are galvanically isolated from other electrodes in that particular array. However, as depicted In the figures, respective electrodes In the receiver array are electrically connected with respective electrodes in the first transmitter array. The amount that the electrodes of the first transmitter array is offset along the first direction with respect to the respective electrodes of the receiver array defines the identification code. Thus, the offset value may be defined by a a given displacement as well as the value "0" which yields the code "0" where the first transmitter array is aligned with the receiver array.

Referring to WO 2007/107562, as shown in Fig. 8, two cartridges 1 and 1' according to embodiments useable with the present invention are depicted. As seen, each of the cartridges comprise a body having a front end 2 and a back end 3. At the front end an arrangement for securing a fluid conduit such as an injection needle (not shown) Is provided. The arrangement shown comprises an adaptor top having a threaded outer surface 4 being adapted to engage with a threaded Inner surface of a mount or hub (not shown) which holds the injection needle or infusion set. In order for a medicament, such as insulin, to be expelled from the cartridge a pierceable membrane may be provided In or near the adaptor top. This pierceable member Is penetrated by an Inwardly oriented needle part when the mount or hub is secured to the adaptor top.

Medicament contained In the cartridge is expelled by displacing a piston 5 In the direction towards the front end 2 of the cartridge. When the piston 5 is moved in the direction of the front end the cartridge, the volume accommodated between the piston and the front end is decreased whereby medicament is forced to leave the cartridge via the fluid conduit attached to the front end of the cartridge.

The enlarged portions of fig. 8 shows exterior surface parts of cartridges carrying identification codes 6 and 6'. The upper cartridge 1 in Fig. 8 carries electrode elements with code "0" whereas the lower cartridge 1' of Fig. 8 carries electrode elements with code "n". The electrode elements may be arranged in various ways, such as for example as an essentially periodic pattern around the exterior surface of the housing. The pattern does not necessarily need to go all the way around the housing. Thus, only part of the 360 degrees around the body of the cartridge may be covered. However, according to a preferred embodiment of the present invention the pattern of electrode elements goes all around the body of the cartridge whereby the reading of the pattern becomes Independent of the angular orientation of the cartridge when Inserted Into the associated medication delivery device.

The electrode elements depicted in Fig. 8 can be mounted or printed directly onto the cartridge as electrically conducting electrodes or pads. Alternatively, the electrode elements can be printed, or by other means provided, directly on a substantially plane, flexible label. The label may have an adhesive material provided on one of its surfaces whereby the label, with the electrode elements arranged or provided thereon, can be positioned on the cartridge. The dimensions of the label are preferably matched to the dimensions of the cartridge. Thus, when the label carrying the electrode elements is arranged on the cartridge by adhesive means, the electrode elements are distributed equally around the complete circumference of the cartridge.

When the cartridge has been Inserted Into an associated medication delivery device (not shown) a number of electrode elements are aligned with a reader head comprising the transmitter/receiver arrangement provided inside the medication delivery device. By aligned is meant that a number of the receiver pads are brought within capacitive coupling distance from the transmitters of the reader head. Similarly, the transmitter pads are brought within capacitive coupling distance from the receivers electrodes of the reader head. Thus, when the cartridge has been Inserted in the medication delivery device the capacitance yielding the particular code and thereby the ID of the cartridge can be determined. When the ID of the cartridge has been determined the type of medicament contained within the cartridge is known. In fact a control unit of the medication delivery device may be configured to accept only certain types of medicament cartridges. Thus, If the content of the cartridge does not belong to this group of medicaments a warning signal can be provided to the user of the medication delivery device informing the user that a medicament of a wrong type has been Inserted Into the medication delivery device. The control unit can also be configured to constantly perform, i.e. as long as the cartridge is positioned in the medication delivery device, a reading of the ID of the cartridge.

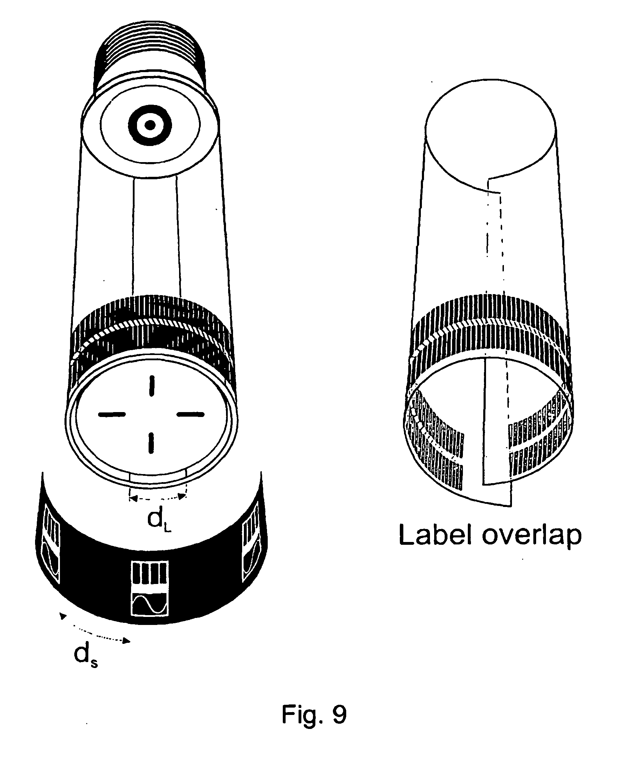

When reading an electronic code printed along an overlapping edge of the label attached to a cylindrically shaped cartridge, a misread of the electronic code may occur If the reader head happens to coincide with the label overlap zone when the cartridge Is Inserted Into the medication delivery device. More than one reader head needs to be used to discriminate against and correct for such code misreads. By placing several (more than one, preferably three) reader heads along the code this problem can be solved. The distance between two adjacent reader heads, **d**ₛ, is greater than the label overlap **d_{L},** see Fig. 9. In this way only one reader head can be affected by the label overlap.

By using three reader heads a majority voting system can be used by the device electronics to first identify the reader head placed wholly or partially over the label overlap and to compare the readings from the other reader heads. If these are reading the same code the readings are deemed to be correct. If the codes are not the same the device electronics will signal a non-read status and, for example, prompt the user to remove and re-insert the cartridge. This situation may arise from label damage or liquid contamination of the cartridge.

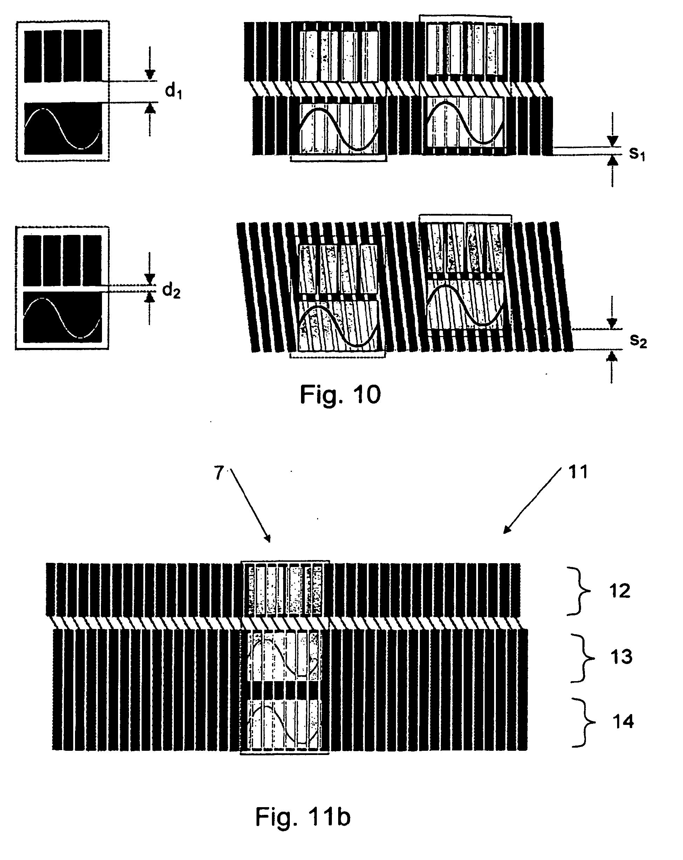

As stated above, the suggested capacitive coding scheme uses separate arrays of electrodes forming transmitter arrays and receiver arrays respectively. The respective electrodes of the two arrays are connected in pairs so that a transmitter electrode is connected to its receiver electrode by means of a thin conductor. This arrangement may limit the range of movement a reader head can have while still having full coverage of the code patterns. By substituting the transmitter-receiver pad pair with an equivalent code comprising a solid slanted bar, see Fig. 10, the reader head may be made more compact In that the transmitter and receiver parts of the reader head may be moved closer together. This more compact design of the reader head is illustrated in Fig. 10, where d₂ < d₁ where d₂ is the distance between the device transmitter electrodes and the receiver electrodes in the compact design (Fig. 10, lower part), whereas d₁ Is the distance between the device transmitter electrodes and the receiver electrodes in the non-compact design (Fig. 10, upper part). The compact reader head can then utilise the whole range of travel perpendicular to the code, thus making the code/reader system less sensitive to misalignments in this dimension (s₂ > s₁).

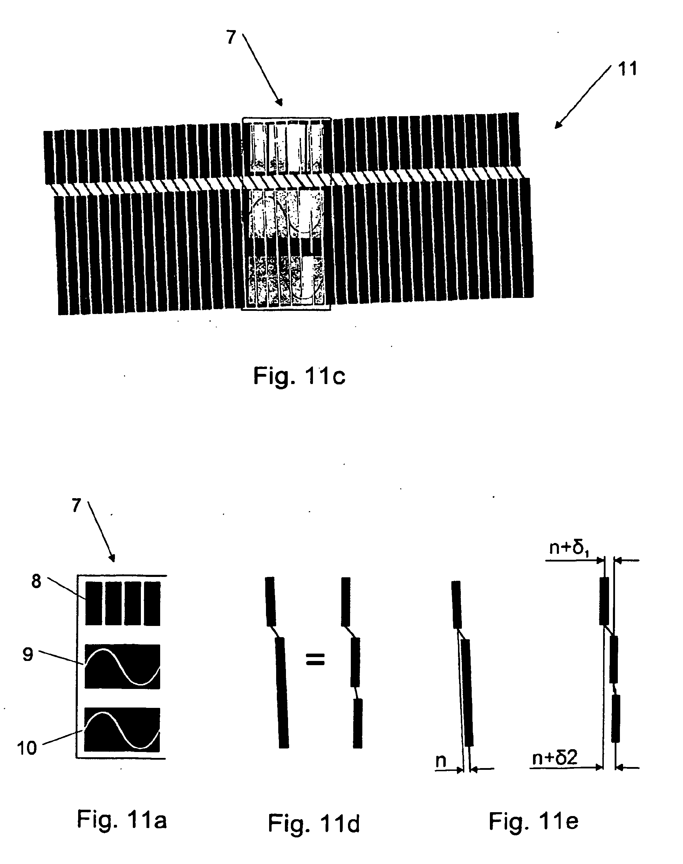

Now turning to Fig. 11 a-e, the principle Is shown where an identification code 11, adapted to be arranged on a medicament reservoir such as a cartridge (not shown), is detectable by a reader head assembly 7 mountable In an associated medical delivery device (not shown). In accordance with the principle described above, the reader head assembly 7 as depicted in Fig. 11a comprises a group of device transmitter electrodes 8 which is connected to further electric circuitry (not shown) of the delivery device. The electric circuitry is adapted to generate a plurality of Input signals each to be applied to respective device transmitter electrodes of the reader head assembly as discussed above. The device transmitter electrodes may be disposed along a second direction so that, in use, the transmitter electrodes are substantially aligned with the receiver array 12 of the identification code 11.

Further, the reader head assembly comprises a first reader head 9 which may be of the same design as discussed above in relation to Fig. 4 to 7. In use, the first reader head 9 is to be substantially aligned with a first transmitter array 13 of the identification code 11. In accordance with the present invention, the reader head assembly further comprise a second reader head 10 which functionally corresponds to the first reader head 9. In use, the second reader head 10 Is to be substantially aligned with a second transmitter array 14 of the identification code 11.

Fig. 11b shows the identification code 11 with the reader head assembly 7 superposed at a specific location of the identification code with the identification code perfectly aligned with the reader head assembly. In the embodiment shown, the electrodes of the second transmitter array 14 are aligned with the respective electrodes of the first transmitter array 13. Each of the electrodes of the first and second transmitter arrays are electrically connected by a region of same width as the width of each of the electrodes. However, in an alternative form, the said electrodes may be connected by a narrow part Interconnecting the two electrodes.

Fig. 11c shows the same arrangement of the identification code 11 and the reader head assembly 7, but in the depicted state the identification code 11 is somewhat skewed relative to the reader head assembly 7. Such misalignment may occur If the identification code is not perfectly aligned with the particular reservoir used. Typically, for cylindrical cartridges provided with a label carrying the identification code, the misalignment may be subject to Inaccurate label positioning. Also, misalignment may occur if the reservoir is held somewhat Inaccurately in the medical delivery device.

In the left-hand part of Fig. 11d, a single electrode pair of electrodes from the identification code 11 in a skewed state relative to the reader head assembly 7 Is depicted. In the righthand part of Fig. 11d, a functionally equivalent electrode configuration is depicted. As shown In Fig. 11e, the specific layout of the code yield an offset value of magnitude n of electrodes In the first and second transmitter arrays with respect to their corresponding electrode In the receiver. Using a reader head assembly which Is skewed with respect to the identification code, a reading of n+δ₁ will be obtained at the first reader head 9, whereas a reading of n+δ₂ will be obtained at the second reader head 10. Using the specific geometric example of the drawings where the electrode of the first transmitter array are arranged half-way between the electrode of the receiver array and the electrode of the second transmitter array, the difference between the code value read by the upper reader head 9, n+δ₁, and the code value read by the lower reader head 10, n+δ₂, can be used to calculate n. With the shown geometries, the correction can be calculated from δ₂ = 2* δ₁. Naturally, if the reading at the upper reader head 9 and the lower reader head 10 is substantially identical, the Identification code is appropriately aligned with the reader head assembly 7.

Using a layout of the identification code and using a corresponding design of the associated reader head assembly which differs from the one above, it will be readily appreciated by the skilled reader that the compensation needs to be adjusted to the particular choice of geometry.

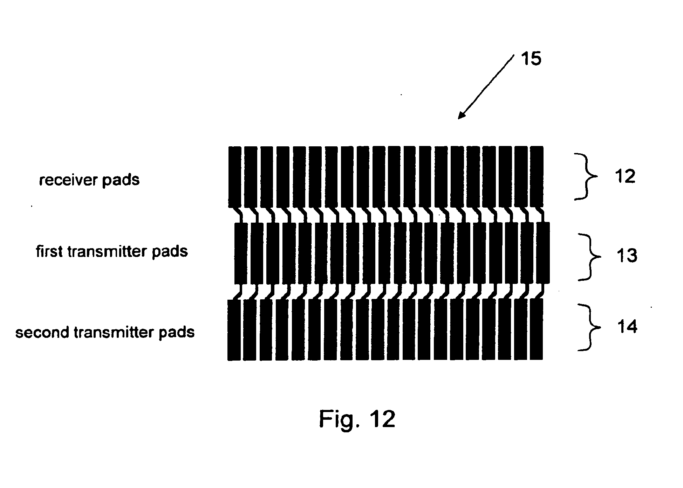

In an embodiment of the invention as shown in Fig. 12, the layout of the identification code 15 is such that electrodes of the second transmitter array 14 are aligned with corresponding electrodes in the receiver array (offset value = 0), while the electrodes of the first transmitter array 13 are offset by a magnitude n corresponding to the specific type code n. Using such a layout, If the reading by the lower reader head equals zero, then the identification code is deemed In perfect alignment with the reader head assembly 7. If the reading is different than zero, then the code value can be estimated by compensating the reading obtained at the upper reader head 9 while taking Into account the skew angle as estimated by the reading obtained at lower reader head 10.

In a still further embodiment, the layout of the identification code is similar to the one shown in Fig. 12. Also In this embodiment, the reader head assembly is similar to the one shown in Fig. 11a. If a sufficient accurate alignment of the identification code with respect to the reading head assembly can be obtained, the two independent transmitter arrays can be used to obtain a system having N*N distinct codes as opposed to the above examples which only may provide N distinct codes. However, in order to additionally compensate for the problem of skewed angles, an additional transmitter track and an additional reading head may be provided In the same way as referred to above.

It should be stressed that any of the features described in connection with figures 3-10 may be used In the design of the coding system according to the present invention.

## Claims

1. A medicament reservoir for a medical delivery device adapted to receive the medicament reservoir and adapted to capacitively couple to and identify an identification code arranged on the medicament reservoir when received by the medical delivery device, the reservoir identification code comprising
- a first array of discrete electrodes having an equally spaced pitch P and forming a receiver array, and
- a second array of discrete electrodes having an equally spaced pitch P and forming a first transmitter array,
wherein the receiver array and the first transmitter array are arranged substantially parallel and extending along a first direction, the respective electrodes of the receiver array being galvanically coupled to respective electrodes of the first transmitter array, and
wherein the electrodes of the first transmitter array are offset along the first direction with respect to the respective electrodes of the receiver array by a first offset value, the magnitude of the first offset value being dedicated the type of medicament reservoir,
wherein a further third array of discrete electrodes having an equally spaced pitch P and forming a second transmitter array is arranged substantially parallel to the first transmitter array, the respective electrodes of the second transmitter array being galvanically coupled to respective electrodes of the first transmitter array and wherein the electrodes of the second transmitter array are offset along the first direction with respect to the respective electrodes of the first transmitter array.

2. A medicament reservoir according to claim 1, wherein the electrodes of the second transmitter array are aligned along the first direction with respect to respective electrodes of the receiver array.

3. A medicament reservoir according to claim 1 or 2, wherein the position of the electrodes of the second transmitter array along the first direction with respect to respective electrodes of the receiver array defines a second offset value, the combination of the first and second offset values being dedicated the type of medicament reservoir.

4. A medicament reservoir according to claim 1-3, wherein the first transmitter array is positioned between the receiver array and the second transmitter array.

5. A medicament reservoir according to claim 1-4, wherein electrodes of the said arrays are arranged on a flexible foil, said foil being secured to an exterior surface part of the medicament reservoir.

6. A medicament reservoir according to claim 1-5,wherein electrodes of the said arrays are at least partly covered by a layer of dielectric material.

7. A medicament reservoir according to claim 1-6, wherein the reservoir comprises a cylindrical portion and wherein the receiver array, the first transmitter array and the second transmitter array extends substantially 360 degrees around the cylindrical portion.

8. A medical delivery device adapted to receive a medicament reservoir, the medicament reservoir having a reservoir identification code comprising
- a first array of discrete electrodes having an equally spaced pitch P and forming a receiver array,
- a second array of discrete electrodes having an equally spaced pitch P and forming a first transmitter array, and
- a third array of discrete electrodes of equally spaced pitch P and forming a second transmitter array,
wherein the receiver array, the first transmitter array and the second transmitter array are arranged substantially parallel and each extending along a first direction, the respective electrodes of the receiver array being galvanically coupled to respective electrodes of the first transmitter array and the second transmitter array, and
wherein the electrodes of the first transmitter array are offset along the first direction with respect to the respective electrodes of the receiver array, thereby defining a first offset value, the magnitude of the first offset value being dedicated the type of medicament reservoir and
wherein the medical delivery device comprises
- a group of device transmitter electrodes of equally spaced pitch P arranged along a second direction which, when a reservoir is received in the device, substantially aligns with said first direction, the device transmitter electrodes being adapted to capacitively couple to a respective group of electrodes of the receiver array disposed on the reservoir,
- a first reader head having electrodes adapted to capacitively couple to a respective group of electrodes of the first transmitter array disposed on the reservoir,
- signal generator means for applying a plurality of time varying input signals to respective electrodes of said group of device transmitter electrodes,
- electric circuitry adapted to receive signals detected by the first reader head to detect the magnitude of a third offset value representing the measured offset of electrodes of the first transmitter array along the first direction with respect to electrodes of the receiver array,
wherein the medical delivery device further comprises a second reader head having electrodes for capacitively coupling to a respective group of electrodes of the second transmitter array disposed on the reservoir, and
wherein the electric circuitry is adapted to receive signals from the second reader head to detect the magnitude of a fourth offset value representing the measured offset of electrodes of the second transmitter array along the first direction with respect to electrodes of the receiver array and to identify the reservoir identification code on the basis of the third and fourth offset values.

9. A medical delivery device according to claim 8, wherein the electric circuitry is adapted to detect a skew angle defined by the angle between the first direction and the second direction based upon the detected fourth offset value.

10. A medical delivery device according to claim 9, wherein the medical delivery device further comprises alarm means coupled to the electric circuitry and that the electric circuitry is adapted to generate an alarm if a detected skew angle is larger than a pre-defined value.

11. A medical delivery device according to claim 9 or 10, wherein the electric circuitry is adapted to calculate a modified code offset value based upon the detected third offset value and compensated by the detected skew angle.

12. A medical delivery device according to claim 8, wherein the electric circuitry is adapted to identify an identification code in accordance with the combination of the detected third and fourth offset values.

13. A medical delivery device according to any of claims 8 to 13 comprising a medicament reservoir according to claims 1 to 7.

14. A flexible foil adapted to be arranged on an exterior surface part of a medicament reservoir for a medical delivery device, the flexible foil having a reservoir identification code which is identifiable by the medical delivery device, the reservoir identification code comprising
- a first array of discrete electrodes having an equally spaced pitch P and forming a receiver array, and
- a second array of discrete electrodes having an equally spaced pitch P and forming a first transmitter array,
wherein the receiver array and the first transmitter array are arranged substantially parallel and extending along a first direction, the respective electrodes of the receiver array being galvanically coupled to respective electrodes of the first transmitter array, and
wherein the electrodes of the first transmitter array are offset along the first direction with respect to the respective electrodes of the receiver array by a first offset value, the magnitude of the first offset value being dedicated the type of medicament reservoir,
wherein a further third array of discrete electrodes having an equally spaced pitch P and forming a second transmitter array is arranged substantially parallel to the first transmitter array, the respective electrodes of the second transmitter array being galvanically coupled to respective electrodes of the first transmitter array and wherein the electrodes of the second transmitter array are offset along the first direction with respect to the respective electrodes of the first transmitter array.

## Patentansprüche

1. Medikamentenbehälter für eine Medikamentenabgabevorrichtung, die angepasst ist zum Aufnehmen des Medikamentenbehälters und angepasst ist zum kapazitiven Koppeln an einem und Erkennen eines auf dem Medikamentenbehälter angeordneten Kennzeichnungscodes, wenn er von der Medikamentenabgabevorrichtung aufgenommen ist, wobei der Behälterkennzeichnungscode umfasst
- eine erste Anordnung von einzelnen Elektroden, die eine abstandsgleiche Teilung P aufweisen und eine Empfängeranordnung bilden, und
- eine zweite Anordnung von einzelnen Elektroden, die eine abstandsgleiche Teilung P aufweisen und eine erste Senderanordnung bilden,
wobei die Empfängeranordnung und die erste Senderanordnung im Wesentlichen parallel angeordnet sind und sich entlang einer ersten Richtung erstrecken, wobei die entsprechenden Elektroden der Empfängeranordnung an entsprechende Elektroden der ersten Senderanordnung galvanisch gekoppelt sind, und
wobei die Elektroden der ersten Senderanordnung entlang der ersten Richtung in Bezug auf die entsprechenden Elektroden der Empfängeranordnung durch einen ersten Versatzwert versetzt sind, wobei die Größenordnung des ersten Versatzwerts dem Medikamentenbehältertyp zugeordnet ist,
wobei eine weitere dritte Anordnung von einzelnen Elektroden, die eine abstandsgleiche Teilung P aufweisen und eine zweite Senderanordnung bilden, im Wesentlichen parallel zu der ersten Senderanordnung angeordnet ist, wobei die entsprechenden Elektroden der zweiten Senderanordnung an entsprechende Elektroden der ersten Senderanordnung galvanisch gekoppelt sind, und wobei die Elektroden der zweiten Senderanordnung entlang der ersten Richtung in Bezug auf die entsprechenden Elektroden der ersten Senderanordnung versetzt sind.

2. Medikamentenbehälter nach Anspruch 1, wobei die Elektroden der zweiten Senderanordnung entlang der ersten Richtung in Bezug auf entsprechende Elektroden der Empfängeranordnung abgeglichen sind.

3. Medikamentenbehälter nach Anspruch 1 oder 2, wobei die Position der Elektroden der zweiten Senderanordnung entlang der ersten Richtung in Bezug auf entsprechende Elektroden der Empfängeranordnung einen zweiten Versatzwert definiert, wobei die Kombination des ersten und des zweiten Versatzwerts dem Medikamentenbehältertyp zugeordnet ist.

4. Medikamentenbehälter nach Anspruch 1-3, wobei die erste Senderanordnung zwischen der Empfängeranordnung und der zweiten Senderanordnung positioniert ist.

5. Medikamentenbehälter nach Anspruch 1-4, wobei Elektroden der Anordnungen auf einer flexiblen Folie angeordnet sind, wobei die Folie auf einem Außenflächenteil des Medikamentenbehälters befestigt ist.

6. Medikamentenbehälter nach Anspruch 1-5, wobei Elektroden der Anordnungen zumindest teilweise von einer Schicht aus dielektrischem Material bedeckt sind.

7. Medikamentenbehälter nach Anspruch 1-6, wobei der Behälter einen zylinderförmigen Abschnitt umfasst und wobei sich die Empfängeranordnung, die erste Senderanordnung und die zweite Senderanordnung im Wesentlichen mit 360 Grad um den zylinderförmigen Abschnitt erstrecken.

8. Medikamentenabgabevorrichtung, die angepasst ist zum Aufnehmen eines Medikamentenbehälters, wobei der Medikamentenbehälter einen Behälterkennzeichnungscode aufweist, der umfasst
- eine erste Anordnung von einzelnen Elektroden, die eine abstandsgleiche Teilung P aufweisen und eine Empfängeranordnung bilden,
- eine zweite Anordnung von einzelnen Elektroden, die eine abstandsgleiche Teilung P aufweisen und eine erste Senderanordnung bilden, und
- eine dritte Anordnung von einzelnen Elektroden, die eine abstandsgleiche Teilung P aufweisen und eine zweite Senderanordnung bilden,
wobei die Empfängeranordnung, die erste Senderanordnung und die zweite Senderanordnung im Wesentlichen parallel angeordnet sind und sich entlang einer ersten Richtung erstrecken, wobei die entsprechenden Elektroden der Empfängeranordnung an entsprechende Elektroden der ersten Senderanordnung und der zweiten Senderanordnung galvanisch gekoppelt sind, und
wobei die Elektroden der ersten Senderanordnung entlang der ersten Richtung in Bezug auf die entsprechenden Elektroden der Empfängeranordnung versetzt sind, wodurch ein erster Versatzwert definiert wird, wobei die Größenordnung des ersten Versatzwerts dem Medikamentenbehältertyp zugeordnet ist, und
wobei die Medikamentenabgabevorrichtung umfasst
- eine Gruppe von Senderelektroden der Vorrichtung mit einer abstandsgleichen Teilung P, angeordnet entlang einer ersten Richtung, die wenn ein Behälter in der Vorrichtung aufgenommen ist, im Wesentlichen an die erste Richtung angeglichen ist, wobei die Senderelektroden der Vorrichtung zum kapazitiven Koppeln an eine entsprechende Elektrodengruppe der auf dem Behälter angeordneten Empfängeranordnung angepasst sind,
- einen ersten Lesekopf mit Elektroden, die zum kapazitiven Koppeln an eine entsprechende Elektrodengruppe der auf dem Behälter angeordneten ersten Senderanordnung angepasst sind,
- ein Signalerzeugungsmittel zum Zuführen einer Mehrzahl von zeitlich variierenden Eingabesignalen zu den entsprechenden Elektroden der Gruppe von Senderelektroden der Vorrichtung,
- einen elektrischen Schaltkreis, der zum Empfangen von durch den ersten Lesekopf erfassten Signalen angepasst ist, um die Größenordnung eines dritten Versatzwerts zu erfassen, der den gemessenen Versatz von Elektroden der ersten Senderanordnung entlang der ersten Richtung in Bezug auf Elektroden der Empfängeranordnung repräsentiert,
wobei die Medikamentenabgabevorrichtung des Weiteren einen zweiten Lesekopf umfasst, der Elektroden zum kapazitiven Koppeln an eine entsprechende Elektrodengruppe der auf dem Behälter angeordneten zweiten Senderanordnung aufweist, und
wobei der elektrische Schaltkreis zum Empfangen von Signalen von dem zweiten Lesekopf angepasst ist, um die Größenordnung eines vierten Versatzwerts zu erfassen, der den gemessenen Versatz von Elektroden der zweiten Senderanordnung entlang der ersten Richtung in Bezug auf Elektroden der Empfängeranordnung repräsentiert, und den Behälterkennzeichnungscode auf der Basis des dritten und vierten Versatzwerts zu erkennen.

9. Medikamentenabgabevorrichtung nach Anspruch 8, wobei der elektrische Schaltkreis zum Erfassen eines durch den Winkel zwischen der ersten Richtung und der zweiten Richtung definierten schiefen Winkels auf der Basis des erfassten vierten Versatzwerts angepasst ist.

10. Medikamentenabgabevorrichtung nach Anspruch 9, wobei die Medikamentenabgabevorrichtung des Weiteren ein an den elektrischen Schaltkreis gekoppeltes Alarmmittel umfasst und der elektrische Schaltkreis zum Erzeugen eines Alarms, wenn ein erfasster schiefer Winkel größer als ein vordefinierter Wert ist, angepasst ist.

11. Medikamentenabgabevorrichtung nach Anspruch 9 oder 10, wobei der elektrische Schaltkreis zum Berechnen eines modifizierten Codeversatzwerts auf der Basis des erfassten dritten Versatzwerts angepasst und durch den erfassten schiefen Winkel kompensiert ist.

12. Medikamentenabgabevorrichtung nach Anspruch 8, wobei der elektrische Schaltkreis zum Erkennen eines Kennzeichnungscodes gemäß der Kombination des erfassten dritten und vierten Versatzwerts angepasst ist.

13. Medikamentenabgabevorrichtung nach einem der Ansprüche 8 bis 13, die einen Medikamentenbehälter nach den Ansprüchen 1 bis 7 umfasst.

14. Flexible Folie, die zum Anordnen auf einem Außenflächenteil eines Medikamentenbehälters für eine Medikamentenabgabevorrichtung angepasst ist, wobei die flexible Folie einen Behälterkennzeichnungscode aufweist, der durch die Medikamentenabgabevorrichtung erkennbar ist, wobei der Behälterkennzeichnungscode umfasst
- eine erste Anordnung von einzelnen Elektroden, die eine abstandsgleiche Teilung P aufweisen und eine Empfängeranordnung bilden, und
- eine zweite Anordnung von einzelnen Elektroden, die eine abstandsgleiche Teilung P aufweisen und eine erste Senderanordnung bilden,
wobei die Empfängeranordnung und die erste Senderanordnung im Wesentlichen parallel angeordnet sind und sich entlang einer ersten Richtung erstrecken, wobei die entsprechenden Elektroden der Empfängeranordnung an entsprechende Elektroden der ersten Senderanordnung galvanisch gekoppelt sind, und
wobei die Elektroden der ersten Senderanordnung entlang der ersten Richtung in Bezug auf die entsprechenden Elektroden der Empfängeranordnung durch einen ersten Versatzwert versetzt sind, wobei die Größenordnung des ersten Versatzwerts dem Medikamentenbehältertyp zugeordnet ist,
wobei eine weitere dritte Anordnung von einzelnen Elektroden, die eine abstandsgleiche Teilung P aufweisen und eine zweite Senderanordnung bilden, im Wesentlichen parallel zu der ersten Senderanordnung angeordnet ist, wobei die entsprechenden Elektroden der zweiten Senderanordnung an entsprechende Elektroden der ersten Senderanordnung galvanisch gekoppelt sind, und wobei die Elektroden der zweiten Senderanordnung entlang der ersten Richtung in Bezug auf die entsprechenden Elektroden der ersten Senderanordnung versetzt sind.

## Revendications

1. Un réservoir de médicament pour un dispositif médical de délivrance adapté pour recevoir le réservoir de médicament et adapté pour se coupler par voie capacitive à un code d'identification et identifier celui-ci, ce code étant disposé sur le réservoir de médicament lorsqu'il est reçu par le dispositif médical de délivrance, le code d'identification du réservoir comprenant :
- un premier réseau d'électrodes discrètes présentant un pas P à espacement constant et formant un réseau récepteur, et
- un second réseau d'électrodes discrètes présentant un pas P à espacement constant et formant un premier réseau émetteur,
dans lequel le réseau récepteur et le premier réseau émetteur sont disposés substantiellement parallèlement et en s'étendant le long d'une première direction, les électrodes respectives du réseau récepteur étant couplées par voie galvanique aux électrodes respectives du premier réseau émetteur, et
dans lequel les électrodes du premier réseau émetteur sont décalées le long de la première direction par rapport aux électrodes respectives du réseau récepteur d'une première valeur de décalage, l'ampleur de la première valeur de décalage étant représentative du type de réservoir de médicament,
dans lequel un troisième réseau supplémentaire d'électrodes discrètes présentant un pas P à espacement constant et formant un second réseau émetteur est disposé substantiellement parallèlement au premier réseau émetteur, les électrodes respectives du second réseau émetteur étant couplées par voie galvanique à des électrodes respectives du premier réseau émetteur et dans lequel les électrodes du second réseau émetteur sont décalées le long de la première direction par rapport aux électrodes respectives du premier réseau émetteur.

2. Un réservoir de médicament selon la revendication 1, dans lequel les électrodes du second réseau émetteur sont alignées le long de la première direction par rapport aux électrodes respectives du réseau récepteur.

3. Un réservoir de médicament selon la revendication 1 ou 2, dans lequel la position des électrodes du second réseau émetteur le long de la première direction par rapport aux électrodes respectives du réseau récepteur définit une seconde valeur de décalage, la combinaison de la première et de la seconde valeur de décalage étant représentative du type de réservoir de médicament.

4. Un réservoir de médicament selon les revendications 1 à 3, dans lequel le premier réseau émetteur est positionné entre le réseau récepteur et le second réseau émetteur.

5. Un réservoir de médicament selon les revendications 1 à 4, dans lequel les électrodes desdits réseaux sont disposées sur une feuille flexible, ladite feuille étant assujettie à une partie de surface extérieure du réservoir de médicament.

6. Un réservoir de médicament selon les revendications 1 à 5, dans lequel les électrodes desdits réseaux sont au moins partiellement recouvertes d'une couche de matériau diélectrique.

7. Un réservoir de médicament selon les revendications 1 à 6, dans lequel le réservoir comprend une partie cylindrique et dans lequel le réseau récepteur, le premier réseau émetteur et le second réseau émetteur s'étendent substantiellement sur 360° autour de la partie cylindrique.

8. Un dispositif médical de délivrance apte à recevoir un réservoir de médicament, le réservoir de médicament possédant un code d'identification de réservoir comprenant :
- un premier réseau d'électrodes discrètes présentant un pas P à espacement constant et formant un réseau récepteur,
- un second réseau d'électrodes discrètes présentant un pas P à espacement constant et formant un premier réseau émetteur, et
- un troisième réseau d'électrodes discrètes de pas P à espacement constant et formant un second réseau émetteur,
dans lequel le réseau récepteur, le premier réseau émetteur et le second réseau émetteur sont disposés substantiellement parallèlement et chacun s'étendant le long d'une première direction, les électrodes respectives du réseau récepteur étant couplées par voie galvanique à des électrodes respectives du premier réseau émetteur et du second réseau émetteur, et
dans lequel les électrodes du premier réseau émetteur sont décalées le long de la première direction par rapport aux électrodes respectives du réseau récepteur, définissant ainsi une première valeur de décalage, l'ampleur de la première valeur de décalage étant représentative du type de réservoir de médicament, et
dans lequel le dispositif médical de délivrance comprend :
- un groupe d'électrodes émettrices de dispositif de pas P à espacement constant disposées le long d'une seconde direction qui, lorsqu'un réservoir est reçu dans le dispositif, s'aligne substantiellement sur ladite première direction, les électrodes émettrices du dispositif étant adaptées pour se coupler par voie capacitive à un groupe respectif d'électrodes du réseau récepteur disposé sur le réservoir,
- une première tête de lecture possédant des électrodes adaptées pour se coupler par voie capacitive à un groupe respectif d'électrodes du premier réseau émetteur disposé sur le réservoir,
- des moyens générateurs de signal pour appliquer une pluralité de signaux d'entrée variant dans le temps à des électrodes respectives dudit groupe d'électrodes émettrices du dispositif,
- des circuits électriques adaptés pour recevoir des signaux détectés par la première tête de lecture pour détecter la grandeur d'une troisième valeur de décalage représentant le décalage mesuré des électrodes du premier réseau émetteur le long de la première direction par rapport aux électrodes du réseau récepteur,
dans lequel le dispositif médical de délivrance comprend en outre une seconde tête de lecture possédant des électrodes pour se coupler par voie capacitive à un groupe respectif d'électrodes du second réseau émetteur disposé sur le réservoir, et
dans lequel les circuits électriques sont adaptés pour recevoir les signaux provenant de la seconde tête de lecture pour détecter la grandeur d'une quatrième valeur de décalage représentant le décalage mesuré des électrodes du second réseau émetteur le long de la première direction par rapport aux électrodes du réseau récepteur et pour identifier le code d'indentification du réservoir sur la base des troisième et quatrième valeurs de décalage.

9. Un dispositif médical de délivrance selon la revendication 8, dans lequel les circuits électriques sont aptes à détecter un angle d'oblicité défini par l'angle formé entre la première direction et la seconde direction sur la base de la quatrième valeur de décalage détectée.

10. Un dispositif médical de délivrance selon la revendication 9, dans lequel le dispositif médical de délivrance comprend en outre des moyens d'alarme couplés aux circuits électriques et où les circuits électriques sont adaptés pour générer une alarme si un angle d'oblicité détecté est supérieur à une valeur prédéfinie.

11. Un dispositif médical de délivrance selon la revendication 9 ou 10, dans lequel les circuits électriques sont adaptés pour calculer une valeur de décalage de code modifiée sur la base de la troisième valeur de décalage détectée compensée par l'angle d'oblicité détecté.

12. Un dispositif médical de délivrance selon la revendication 8, dans lequel les circuits électriques sont adaptés pour identifier un code d'identification en fonction de la combinaison des troisième et quatrième valeurs de décalage détectées.

13. Un dispositif médical de délivrance selon l'une des revendications 8 à 12, comprenant un réservoir de médicament selon les revendications 1 à 7.

14. Une feuille flexible adaptée pour être disposée sur une partie de surface extérieure d'un réservoir de médicament pour un dispositif médical de délivrance, la feuille flexible possédant un code d'identification de réservoir qui est identifiable par le dispositif médical de délivrance, le code d'identification de réservoir comprenant :
- un premier réseau d'électrodes discrètes présentant un pas P à espacement constant et formant un réseau récepteur, et
- un second réseau d'électrodes discrètes présentant un pas P à espacement constant et formant un premier réseau émetteur,
dans lequel le réseau récepteur et le premier réseau émetteur sont disposés substantiellement parallèlement et en s'étendant le long d'une première direction, les électrodes respectives du réseau récepteur étant couplées par voie galvanique aux électrodes respectives du premier réseau émetteur, et
dans lequel les électrodes du premier réseau émetteur sont décalées le long de la première direction par rapport aux électrodes respectives du réseau récepteur d'une première valeur de décalage, l'ampleur de la première valeur de décalage étant représentative du type de réservoir de médicament,
dans lequel un troisième réseau supplémentaire d'électrodes discrètes présentant un pas à espacement constant (P) et formant un second réseau émetteur est disposé substantiellement parallèlement au premier réseau émetteur, les électrodes respectives du second réseau émetteur étant couplées par voie galvanique à des électrodes respectives du premier réseau émetteur et dans lequel les électrodes du second réseau émetteur sont décalées le long de la première direction par rapport aux électrodes respectives du premier réseau émetteur.
